# EUROPÄISCHE PATENTANMELDUNG

(11) **EP 1 716 756 A1**
(43) Veröffentlichungstag der Anmeldung: **02.11.2006**
(21) Anmeldenummer: 05009471.3
(22) Anmeldetag: 29.04.2005
(51) Int. Cl.: A23D 9/02, A61K 31/202, A23D 9/00, A23L 1/22, A23L 1/30

(54) **Nahrungsergänzungsmittel oder functional food enthaltend Öl-Kombination**

(71) Anmelder: Vinorica S.L., 07330 Consell Mallorca (ES)
(72) Erfinder: Popp, Michael A., 91207 Lauf / Pegnitz (DE)
(74) Vertreter: Vossius & Partner

(57) **Zusammenfassung**

Die vorliegende Erfindung betrifft ein Nahrungsergänzungsmittel oder Functional Food enthaltend oder bestehend aus (a) ein Nussöl in einer Menge von 0,5 bis 20 Volumenprozent; (b) eine ω-3-fettsäurenhaltige Quelle in einer Menge von 10 bis 50 Volumenprozent, wobei die Gesamtmenge von ω-3-Fettsäuren mindestens 20 Volumenprozent beträgt; und (c) einen Geschmacksträger in einer Menge von 10 bis 89,5 Volumenprozent. Weiterhin betrifft die vorliegende Erfindung Salatöl bestehend aus oder enthaltend das erfindungsgemäße Nahrungsergänzungsmittel oder Functional Food. Schließlich betrifft die vorliegende Erfindung die Verwendung des erfindungsgemäßen Nahrungsergänzungsmittels oder Functional Food als Salatöl.

## Beschreibung

Die vorliegende Erfindung betrifft ein Nahrungsergänzungsmittel oder Functional Food enthaltend oder bestehend aus (a) ein Nussöl in einer Menge von 0,5 bis 20 Volumenprozent; (b) eine ω-3-fettsäurenhaltige Quelle in einer Menge von 10 bis 50 Volumenprozent, wobei die Gesamtmenge von ω-3-Fettsäuren mindestens 20 Volumenprozent beträgt; und (c) einen Geschmacksträger in einer Menge von 10 bis 89,5 Volumenprozent. Weiterhin betrifft die vorliegende Erfindung Speiseöl bestehend aus oder enthaltend das erfindungsgemäße Nahrungsergänzungsmittel oder Functional Food. Schließlich betrifft die vorliegende Erfindung die Verwendung des erfindungsgemäßen Nahrungsergänzungsmittels oder Functional Food als Speiseöl.

Herz-Kreislauf-Erkrankungen stehen in Deutschland und Europa an der Spitze der Todesursachenstatistik. Hinter diesem Sammelbegriff verbergen sich verschiedene Krankheitsbilder wie Herzinfarkt, plötzlicher Herztod und Angina pectoris. Oft spricht man in diesem Zusammenhang auch von der sogenannten Koronaren Herzkrankheit, abgekürzt als KHK bezeichnet. Die gemeinsame Ursache all dieser häufigen Erkrankungen ist oftmals eine Verkalkung der Arterien, die sich über viele Jahre unbemerkt entwickelt. Nach und nach verengen sich dadurch die Blutgefäße, das Blut kann nicht mehr ungehindert fließen. So wird das Herz nicht mehr optimal durchblutet. Im schlimmsten Fall kommt es zu einer plötzlichen Verstopfung des Gefäßes, als Thrombose bezeichnet, wodurch die Blut- und Sauerstoffversorgung des Herzens ganz unterbrochen wird. Die Folge ist ein Herzinfarkt.

Zahlreiche wissenschaftliche Untersuchungen haben nachgewiesen, dass falsche Ernährungsgewohnheiten eine der Hauptursachen für diese Risikofaktoren sind. Das bedeutet, dass durch eine entsprechende Umstellung der Ernährung die Entstehung einer Arterienverkalkung hinausgezögert oder sogar ganz verhindert werden kann. Im Zusammenhang mit der Ernährung spielen neben den Vitaminen vor allem die Menge und Art der verzehrten Fette eine entscheidende Rolle für die Entwicklung und das Fortschreiten von Herz-Kreislauf-Erkrankungen. Die richtige Balance zwischen gesättigten Fettsäuren tierischen Ursprungs und ungesättigten Fettsäuren aus Pflanzen und Fischen gewinnt dabei zunehmend an Bedeutung. Von besonderem Interesse sind die sogenannten ω-3-Fettsäuren und ω-6-Fettsäuren. ω-6-Fettsäuren sind in vielen pflanzlichen Ölen zu finden und werden daher bei unseren derzeitigen Ernährungsgewohnheiten in ausreichenden Mengen verzehrt. Die Linolsäure, der wichtigste Vertreter dieser Familie, kommt beispielsweise reichlich in Saflor-, Sonnenblumen-, Soja-, Mais- und Weizenkeimöl, aber auch in Margarine vor. Mehrfach ungesättigte Fettsäuren vom Typ w-3 sind dagegen, zumindest in hohen Konzentrationen, lediglich in bestimmten fetten Fischarten und in Algen enthalten.

ω-3- und ω-6-Fettsäuren zählen zu den langkettigen mehrfach ungesättigten Fettsäuren. Das heißt, sie bestehen aus mindestens 18 Kohlenstoffatomen (C) und haben mehr als eine Doppelbindung. Das w bzw. n steht dafür, dass die Kette der Fettsäuren von der Methylgruppe her nummeriert wird. Die Angabe 3 bzw. 6 bezeichnet die Position der ersten Doppelbindung am dritten bzw. sechsten Kohlenstoffatom. Da der menschliche Organismus beide Fettsäuren nicht aus anderen Fettsäuren aufbauen kann, gelten die w-3-Fettsäure und die ω-6-Fettsäure als essentiell. Aus diesen beiden Fettsäuren kann der Körper mit Hilfe der Enzyme Desaturase und Elongase in begrenzten Mengen die längerkettigen Verbindungen aufbauen, die er für verschiedene Stoffwechselfunktionen benötigt.

Eine Reihe von Eigenschaften der w-3-Fettsäuren tragen zur vorbeugenden Wirkung vor Herzinfarkt bei: Studien an Gesunden und Kranken haben gezeigt, dass eine hohe Aufnahme an ω-3-Fettsäuren die Triglycerid- und Cholesterinwerte im Blut reduzieren kann. Eine deutliche Senkung der Blutfettwerte trat allerdings erst bei einer relativ hohen Aufnahme von 3-4 Gramm pro Tag auf. Die ω-3-Fettsäuren reduzieren insbesondere das ungünstige LDL-Cholesterin. Forschern ist zudem aufgefallen, dass das Blut der Inuiten langsamer gerinnt als das der Europäer. Für Personen, die Herzinfarkt gefährdet sind, ist dies erwünscht. Denn vielfach sind es Blutgerinnsel, die die verengten Adern verstopfen und so einen Infarkt auslösen. ω-3-Fettsäuren bewirken, dass weniger Substanzen gebildet werden, die die Blutgerinnung fördern.

Gleichzeitig dienen ω-3-Fettsäuren als Ausgangsprodukt für Stoffe, die die Blutgefäße erweitern, so dass sich Gerinnsel nicht so leicht festsetzen können. Werden ω-3-Fettsäuren vermehrt in die Zellwände eingebaut, sind zudem die roten Blutkörperchen elastischer, was ebenfalls einen ungestörten Blutfluss fördert. Diese Eigenschaften scheinen sich auch günstig auf den Blutdruck auszuwirken.

Bereits seit vielen Jahren ist bekannt, dass Fettsäuren auch bestimmte Immunfaktoren und Entzündungsprozesse beeinflussen. In klinischen Studien wird daher ihr Effekt auf chronisch entzündliche Erkrankungen wie Colitis Ulcerosa, Morbus Crohn, Rheuma und Mukoviszidose untersucht. Ungesättigte Fettsäuren sind Bausteine für Gewebshormone, die auf den Ablauf von Entzündungen einwirken. Während die aus der ω-6-Fettsäure gebildeten Hormone Entzündungen eher fördern, wirken Prostaglandine und Leukotriene, die aus der ω-3-Fettsäure EPA produziert werden, entzündungshemmend. Für Patienten, die an einer entzündlichen Erkrankung leiden, scheint es daher günstig zu sein insbesondere vermehrt ω-3-Fettsäuren aufzunehmen.

Bei Patienten mit Dickdarmkrebs, die täglich Fischölkonzentrate mit zwei bis sieben Gramm ω-3-Fettsäuren schluckten, teilten sich die Krebszellen langsamer als bei einer Vergleichsgruppe. Eine tumorhemmende Wirkung ließ sich aber nur nachweisen, wenn der Gesamtfettgehalt der Nahrung nicht mehr als 30 Prozent der Energiezufuhr betrug. Neben anderen Maßnahmen empfehlen die Experten daher, zur Krebsvorbeugung auf ein ausgewogenes Verhältnis von w-6- zu w-3-Fettsäuren und auf eine niedrige Gesamtfettzufuhr zu achten.

Weiterhin wird in der Literatur eine vorteilhafte Wirkung von ω-3 Fettsäuren bei Rheumatoider Arthritis diskutiert. (Rennie et al. "Nutritional management of rheumatoid arthritis: a review of the evidence", J. Hum. Nutr. Dietet. 16, (2003), Seiten 97-109). Dabei wird auf Dokumentationen verwiesen, die einen positiven Effekt von langkettigen n-3 vielfach ungesättigten Fettsäuren auf die Symptome darstellen. Es wird eine Diät empfohlen, die reich an langkettigen n-3 PUFA und Antioxidantien ist. Des weiteren wird ausgeführt, dass mit anderen Ölen keine signifikante Verbesserung gesehen würde.

Eine weitere Studie befasst sich mit den Effekten von verschiedenen Ölen auf die Gesundheit, insbesondere auf Herzerkrankungen (Katan et al., "Dietary oils, serum lipoproteins, and coronary heart disease", Am. J. Clin. Nutr. 61 (1995), 1368-1373). Dabei wird die Bevölkerung von Kreta als Beispiel einer Bevölkerung hervorgehoben, die auf der Basis einer hohen Einnahme von Olivenöl als Fettquelle eine niedrige Häufigkeit an Herzerkrankungen aufwies. Auch diese Studie bestätigt, dass gesättigte Fettsäuren im Prinzip LDL-Cholesterin erhöhen und damit die Anfälligkeit für Herzerkrankungen. Dagegen setzen ungesättigte Fettsäuren dieses Risiko herab. Es wird ferner beschrieben, dass Fischöle LDL und Apoprotein B im Blut erhöhen können, wobei das gleiche für fetten Fisch gilt. Allerdings haben Fischöl und fetter Fisch vorteilhafte Auswirkungen auf die Serumtriglyceride sowie "very-low-density lipoprotein". Dennoch gilt als erwiesen, dass Fischöle in Populationen, die viel Fischöl zu sich nehmen, die Häufigkeit an Herzerkrankungen herabsetzt.

Eine weitere Publikation diskutiert die Wirksamkeit von pflanzlichen und marinen ω-3 Fettsäuren in der Prävention und Behandlung verschiedener Erkrankungen wie Herzkreislauferkrankungen und Schlaganfall (Kris-Etherton et al., "Fish Consumption, Fish Oil, ω-3 Fatty Acids, and Cardiovascular Disease", Circulation 106 (2002), p. 2747-2757). Im wesentlichen werden groß angelegte Studien diskutiert, die überwiegend belegen, dass diese Fettsäuren einen positiven/präventiven Effekt auf Krankheitsverläufe/Krankheitsausbrüche haben. Allerdings ist bei einigen Studien auch keine Wirksamkeit nachgewiesen worden. Während Fischöl und fetter Fisch als Hauptquellen für marine w-3 Fettsäuren (insbesondere EPA und DHA) genannt werden, werden bei den pflanzlichen Produkten Olivenöl, Rapsöl, Walnussöl und Leinsamenöl genannt, die nicht ganz so hohe Konzentrationen enthalten.

Darlington et al. ("Antioxidantien and fatty acids in the amelioration of rheumatoid arthritis and related disorders", British Journal of Nutrition, 85 (2001), 251-269) diskutieren die Möglichkeiten zur Behandlung von RA und ähnlichen Krankheiten wie Lupus erythromatosis. Während die Autoren darauf verweisen, dass verfügbaren Studien aufgrund der Versuchsbedingungen nicht 100%ig aussagekräftig sind, wird dennoch deutlich, dass langkettige ω-3 Fettsäuren einen positiven Einfluss auf die Krankheit haben. In Zusammenhang mit der Gabe von Fischöl wird ausgeführt, dass dieses zusammen mit einem Anti-Oxidans gegeben wird. Weiterhin wird ausgeführt, dass die gemeinsame Gabe von EPA und DHA wirksamer ist als die Gabe einer dieser ungesättigten Fettsäuren alleine. Schließlich berichten die Autoren davon, dass ein marines Extrakt von Perna canaliculus, der reich an biologisch-aktiven Ölen und natürlichen Anti-Oxidantien war, neben einem therapeutischen auch eine protektiven Effekt gegenüber entzündlicher Arthritis in Ratten aufweist.

Insgesamt zeigt sich also, dass in bestimmten Lebenssituation, oder auch generell, eine vermehrte Aufnahme von ω-3 Fettsäuren wünschenswert wäre. Besonders vorteilhafte Quellen von ω-3 Fettsäuren sind Algen und bestimmte Fischarten. Im Stand der Technik ist jedoch bekannt, dass die Aufnahme von w-3 Fettsäuren mit der Wahrnehmung eines als unangenehm empfundenen Fischgeschmacks verbunden wird. Aus diesem Grunde kommt der Formulierung der ω-3 Fettsäuren in Form von Kapseln zur Zeit noch eine herausragende Bedeutung zu, da bei dieser Art der Aufnahme von w-3 Fettsäuren der unangenehme Geruch oftmals kaum wahrnehmbar, wenn auch bei Aufstoßen nicht zu vermeiden ist. Eine Verwendung, beispielsweise in Form eines Speiseöls mit hohem Anteil an ω-3 Fettsäuren ist jedoch aus den genannten Gründen ausgeschlossen.

Der Erfindung lag somit die Aufgabe zugrunde, ein Nahrungsergänzungsmittel oder Functional Food bereitzustellen, das die vorstehend erörterten Probleme löst und auch als Grundlage für ein Speiseöl eingesetzt werden kann.

Die Lösung der Aufgabe erfolgt durch die in den Ansprüchen gekennzeichneten Ausführungsformen.

Somit betrifft die vorliegende Erfindung ein Nahrungsergänzungsmittel oder Functional Food enthaltend oder bestehend aus (a) ein Nussöl in einer Menge von 0,5 bis 20 Volumenprozent; (b) eine ω-3-fettsäurenhaltige Quelle in einer Menge von 10 bis 50 Volumenprozent, wobei die Gesamtmenge von ω-3-Fettsäuren mindestens 20 Volumenprozent beträgt; und (c) einen Geschmacksträger in einer Menge von 10 bis 89,5 Volumenprozent.

"Nahrungsergänzungsmittel" sind im allgemeinen Lebensmittel, die einen oder mehrere Nährstoffe in konzentrierter Form enthalten. Sie umfassen in lebensmitteluntypischer Form üblicherweise Kapseln, Pastillen, Tabletten, Flüssigkeiten. Weitere typische Darreichungsformen sind Pulverbeutel, Flüssigampullen, Flaschen mit Tropfeinsätzen und ähnlichen Darreichungsformen von Flüssigkeiten und Pulvern zur Aufnahme in abgemessenen kleinen Mengen, die der Ergänzung der Ernährung dienen sollen. Erfindungsgemäß bevorzugt ist eine flüssige/ölige oder streichfähige Konsistenz.

Als "Functional Food" werden erfindungsgemäß Lebensmittel und entsprechende neuentwickelte Produkte zusammengefasst, denen aufgrund besonderer Inhaltsstoffe mehr als nur der reine Nähr- und Geschmackswert zukommt. Erfindungsgemäß geht es um die mittel- oder langfristige Erhaltung und Förderung der Gesundheit. Synonym, teilweise aber auch differenziert, werden die Begriffe Nutriceuticals, Foodsceuticals und Designer Foods verwendet, die ebenfalls Ausführungsformen gemäß der Erfindung darstellen. Der präventive Aspekt und die Förderung der Gesundheit sowie der Lebensmittelcharakter der Produkte werden aber am besten durch den Begriff Functional Food deutlich gemacht. In vielen Fällen handelt es sich um Produkte, die durch Auswahl und Selektion (wie auch im Falle der vorliegenden Erfindung), Reinigung, Konzentration, zunehmend auch durch Addition angereichert wurden. Isolierte Wirksubstanzen, zumal in Tabletten- oder Pillenform, werden nicht dazugezählt.

Der Begriff "ω-3-Fettsäuren" (omega-3 Fettsäuren) bezeichnet eine für den Menschen wichtige Fettsäurefamilie, die einen bestimmten Stoffwechselweg kennzeichnet. Dazu gehören beispielsweise Linolensäure, Eicosapentaensäure und Docosahexaenoicsäure. Die ω-3-Fettsäure kann in Form eines Öls zugesetzt werden oder aus einer anderen Quelle stammen. In jedem Fall ist zu beachten, dass die Gesamtmenge von ω-3-Fettsäuren mindestens 20 Volumenprozent, z.B. mindestens 30 Volumenprozent wie mindestens 40 Volumenprozent beträgt.

Der Begriffe "Geschmacksträger" bezeichnet allgemein hin einen Stoff, der maßgeblich den wahrgenommen Geschmack eines Nahrungsmittels beeinflusst oder der maßgeblich zum geschmacklichen Charakter des Nahrungsmittels beiträgt.

Die vorstehend genannten Prozentangaben ergänzen sich zu 100%, wenn das Nahrungsergänzungsmittel oder Functional Food aus den Komponenten besteht. Bevorzugt ist, dass die maximale Menge an der ω-3 fettsäurehaltigen Quelle 45%, wie 40% oder 35% beträgt.

Aus epidemiologischen Untersuchungen geht hervor, dass ca. 0,2-0,5g ω-3 Fettsäuren pro Tag günstige Wirkungen hervorrufen. Daher dürften nach heutiger Kenntnis zur Vorbeugung ungefähr 0,5g Fischöl (mit 30% ω-3 Fettsäuren) ausreichend sein, während man bei bestehenden Herzerkrankungen oder entzündlichen Erkrankungen (z.B. Rheuma, Psoriasis etc.) diese Dosis auf ca. 1,5-2,5 g/Tag (ca. 20g/Woche) steigen kann.

Die vorliegenden Bereichsdaten der Tabelle 1 sind aufgrund von Verkostungsversuchen entstanden. Sie belegen, dass das erfindungsgemäße Nahrungsergänzungsmittel oder Functional Food, trotz hoher Konzentrationen von w-3-Fettsäuren (bis zu 50 Volumenprozent), nicht mit der Wahrnehmung eines als unangenehm empfundenen Fischgeruchs verbunden wird. Mit dem erfindungsgemäßen Nahrungsergänzungsmittel oder Functional Food wird überraschenderweise also erreicht, dass durch die Zugabe von Nussöl der "fischige" Geschmack der ω-3 Fettsäure verloren geht, nicht aber der Geschmack des Pflanzenöls verloren geht. Dadurch bekommt das erfindungsgemäße Nahrungsergänzungsmittel oder Functional Food, welches aufgrund des erheblichen Anteils an w-3 Fettsäuren einen hohen gesundheitlichen Wert hat, den Geschmack eines ganz normalen Speiseöls. In einigen Ausführungsformen dient ein Nussöl (auch) als Geschmacksträger, z.B. in Fällen, in denen zwei Nussöle zugegeben werden (vgl. Tabelle 1).

Die Schwelle, ab der ein "fischiger" Geschmack in der erfindungsgemäßen Kombination überhaupt noch wahrgenommen wird liegt, personenabhängig, immerhin bei etwa 50% ω-3 Fettsäuren. Ab einem Ölgehalt von 60% lassen sich in den genannten sensorischen Tests keine individuellen Wahrnehmungsunterschiede mehr feststellen, d.h. auch in der Kombination der genannten Komponenten kommt dann der fischige Geschmack durch.

In einer bevorzugten Ausführungsform wird das Nussöl ausgewählt aus der Gruppe bestehend aus Haselnussöl, Mandelöl, Walnussöl, Pekannussöl, Paranussöl, Cashewnussöl, Pistazienöl, Makadamiaöl, Pinienkernöl. Besonders bevorzugt sind hierbei Haselnussöl, Mandelöl und Walnussöl.

In einer anderen bevorzugten Ausführungsform ist die ω-3-fettsäurenhaltige Quelle des erfindungsgemäßen Nahrungsergänzungsmittel oder Functional Foods ein Öl, wie ein natürlicherweise vorkommendes Öl.

In einer besonders bevorzugten Ausführungsform der Erfindung ist das Öl ein Fischöl, ein Algenöl, oder ein aus Planktonmasse hergestelltes Öl, wobei die Planktonmasse vorzugsweise im wesentlichen oder ausschließlich aus Crypthecodinium cohnii besteht. Der Begriff "im wesentlichen" ist an dieser Stelle dergestalt zu verstehen, dass bevorzugt zumindest 50%, stärker bevorzugt zumindest 75%, noch stärker bevorzugt zumindest 90% und an stärksten bevorzugt zumindest 99% der Planktonmasse aus Organismen der Spezies Crypthecodinium cohnii besteht. Bevorzugt werden Fischöle aus fettreichen Kaltwasserfischen wie Hering, Thunfisch, Makrele oder Lachs gewonnen, sind aber auch aus Sardinen, Forellen, Heilbutt, Dorschen, Kabeljau, Schellfisch, Welsen, Flunder und anderen Fischen nach üblichen Verfahren erhältlich. Weiterhin bevorzugt ist die Gewinnung von ω-3 Fettsäuren aus Algen bzw. Algenöl. Bevorzugte Algen sind beispielsweise Mikroalgen, insbesondere der Familien der Braun-, Rot-, Grün- oder Blaugrünalgen. Hiervon sind besonders bevorzugt die Algen aus der Familie der Phaeoophyceae oder Rhodophyceae. Weiterhin bevorzugt sind Aphanizomenon flos-aquae (AFA-Alge), Spirulina, wobei die Mikroalgen Ulkenia sp. und Schizochytrium besonders bevorzugt sind.

In einer anderen bevorzugten Ausführungsform der Erfindung sind die ω-3-Fettsäuren der Quelle Linolensäure, Eicosapentaensäure und/oder Docosahexaensäure, wobei die *cis*-9,12,15 - Octadecatriensäure, *cis*-5,8,11,14,17-Eicosapentaensäure und *cis-*4,7,10,13,16,19-Docosahexaensäure besonders bevorzugt sind.

In einer weiteren bevorzugten Ausführungsform der Erfindung ist der Geschmacksträger ein Pflanzenöl. In besonderen erfindungsgemäßen Ausführungsformen kann der Geschmacksträger auch ein Nussöl sein.

In einer besonders bevorzugten Ausführungsform ist das Pflanzenöl Olivenöl, Rapsöl, Leinöl, Sojaöl, Weizenkeimöl, Haselnussöl, Walnussöl oder Mandelöl. Rapsöl galt lange Zeit aufgrund des relativ hohen Gehalts an Erucasäure als gesundheitlich als bedenklich. Durch Neuzüchtungen, besonders in Kanada und Westeuropa, ist es jedoch gelungen, den Erucasäuregehalt des Öles auf weniger als 1% zu verringern. Entsprechend sollte das erfindungsgemäße Nahrungsergänzungsmittel oder Functional Food möglichst geringe Mengen an Erucasäure enthalten, vorzugsweise im Bereich von weniger als 1 %.

In einer bevorzugten Ausführungsform enthält das erfindungsgemäße Nahrungsergänzungsmittel oder Functional Food weiterhin ein Antioxidans.

In einer besonders bevorzugten Ausführungsform der Erfindung ist das Antioxidans Tocopherol (Vitamin E, E 306) oder ein Antioxidans ausgewählt aus der Gruppe bestehend aus L-Ascorbinsäure (Vitamin C, E 300), Natrium-L-Ascorbat (E 301), Calcium-L-Ascorbat (E 302), Milchsäure (E 270), Alpha-Tocopherol (E 307), Zitronensäure (E 330). Insbesondere bevorzugt ist das Antioxidans Tocopherol (Vitamin E, E 306). Erfindungsgemäß sind auch Kombinationen dieser Antioxidantien möglich. Sofern ein Antioxidans zugegeben wird, addiert sich dessen Menge zu den übrigen erfindungswesentlichen Komponenten, die sich in diesem Falle nicht mehr zu 100% addieren können (sondern nur zu einem Wert unter 100%).

Dem Fachmann sind zahlreiche künstliche und natürliche Antioxidantien bekannt, die mit wenigen Ausnahmen in dem erfindungsgemäßen Nahrungsergänzungsmittel oder Functional Food verwendet werden können. Hierzu gehören beispielsweise alle zur Vitamin-E-Familie gezählten Antioxidantion, die hauptsächlich in Nüssen und Sonnenblumenkernen enthalten sind. Sie werden meist auch in Pflanzenölen, Margarine und Kakaoprodukten verwendet. Ein anderes Beispiel ist das zur Gruppe der Carotinoide zählende Lycopin, ein weiteres natürlich vorkommendes Antioxidationsmittel, das hauptsächlich in roten Tomaten enthalten ist (ca. 20 mg/kg). Lycopin gehört zu den stärksten natürlichen Oxidationsschutzstoffen. Studien haben gezeigt, dass Lycopin auch als wirksame Substanz zur Verringerung des Risikos für bestimmte Krebsarten dienen kann.

Weiterhin betrifft die vorliegende Erfindung ein Speiseöl und bevorzugt ein Salatöl bestehend aus oder enthaltend das erfindungsgemäße Nahrungsergänzungsmittel oder Functional Food.

Der Begriff "Speiseöl" betrifft jedwede Zubereitung unter Verwendung des erfindungsgemäßen Nahrungsergänzungsmittels oder des erfindungsgemäßen Functional Food, die einer Speise als Öl zugesetzt werden kann. Ein Beispiel hierfür ist die Verwendung eines Speiseöls als Aufstrich oder Tunke für Weißbrot, z.B. bei Weinverkostungen. Das Speiseöl kann neben dem erfindungsgemäßen Nahrungsergänzungsmittel oder Functional Food weitere Geschmackskomponenten wie z.B. Kräuter enthalten.

Schließlich betrifft die vorliegende Erfindung die Verwendung des erfindungsgemäßen Nahrungsergänzungsmittels oder Functional Food als Speiseöl. In einer bevorzugten Ausführungsform der Erfindung ist das Speiseöl ein Salatöl.

### Das Beispiel erläutert die Erfindung:

Die in Tabelle 1 dargestellten Kombinationen sind vorteilhafte Ausführungsformen der Erfindung. Die Zahlenwerte stellen Volumenprozentwerte dar.

**Tabelle 1: Abgebildet sind diejenigen Mischungsverhältnisse der Ölkomponenten des erfindungsgemäßen Nahrungsergänzungsmittel oder Functional Foods, die in einer sensorischen Prüfung der entsprechenden Nahrungsergänzungsmittel oder Functional Food keinen "fischigen" Geschmack aufwiesen.**

| Ifd. Nr | Olivenöl | Rapsöl | Leinöl | Sojaöl | Weizenkeimöl | Haselnussöl | Walnussöl | Mandelöl | Fischöl, andere Quellen and ω- 3-Fettsäuren | Anzahl der Kombipartner |
|---|---|---|---|---|---|---|---|---|---|---|
| | 1 | 2 | 3 | 4 | 5 | 6 | 7 | 8 | 9 | 10 |
| 1 | 10 - 89,5 | -- | -- | -- | -- | -- | -- | 0,5 - 20 | 10 - 50 | 3 |
| 2 | -- | 10 - 89,5 | -- | -- | -- | -- | -- | 0,5 - 20 | 10 - 50 | 3 |
| 3 | -- | -- | 10 - 89,5 | -- | -- | -- | -- | 0,5 - 20 | 10 - 50 | 3 |
| 4 | -- | -- | -- | 10 - 89,5 | -- | -- | -- | 0,5 - 20 | 10 - 50 | 3 |
| 5 | -- | -- | -- | -- | 10 - 89,5 | -- | -- | 0,5 - 20 | 10 - 50 | 3 |
| 6 | -- | -- | -- | -- | -- | 10-89,5 | -- | 0,5 - 20 | 10 - 50 | 3 |
| 7 | -- | -- | | | | -- | 10 - 89,5 | 0,5 - 20 | 10 - 50 | 3 |
| 14 | | | | | | | 0,5 - 20 | 10 - 89,5 | 10 - 50 | 3 |
| 8 | 10 - 89,5 | -- | -- | -- | -- | -- | 0,5 - 20 | -- | 10 - 50 | 3 |
| 9 | -- | 10 - 89,5 | -- | -- | -- | -- | 0,5 - 20 | -- | 10 - 50 | 3 |
| 10 | -- | -- | 10 - 89,5 | -- | -- | -- | 0,5 - 20 | -- | 10 - 50 | 3 |
| 11 | -- | -- | -- | 10 - 89,5 | -- | -- | 0,5 - 20 | -- | 10 - 50 | 3 |
| 12 | -- | -- | -- | -- | 10 - 89,5 | -- | 0,5 - 20 | -- | 10 - 50 | 3 |
| 13 | -- | -- | -- | -- | -- | 10 - 89,5 | 0,5 - 20 | | 10 - 50 | 3 |
| 20 | -- | -- | -- | -- | -- | 0,5 - 20 | 10 - 89,5 | -- | 10 - 50 | 3 |
| 15 | 10 - 89,5 | -- | -- | -- | -- | 0,5 - 20 | -- | -- | 10 - 50 | 3 |
| 16 | -- | 10 - 89,5 | -- | -- | -- | 0,5 - 20 | -- | -- | 10 - 50 | 3 |
| 17 | -- | -- | 10 - 89,5 | -- | -- | 0,5 - 20 | -- | -- | 10 - 50 | 3 |
| 18 | -- | -- | -- | 10 - 89,5 | -- | 0,5 - 20 | -- | -- | 10 - 50 | 3 |
| 19 | -- | -- | -- | -- | 10 - 89,5 | 0,5 - 20 | -- | -- | 10 - 50 | 3 |

## Patentansprüche

1. Nahrungsergänzungsmittel oder Functional Food enthaltend oder bestehend aus
(a) ein Nussöl in einer Menge von 0,5 bis 20 Volumenprozent;
(b) eine ω-3-fettsäurenhaltige Quelle in einer Menge von 10 bis 50 Volumenprozent, wobei die Gesamtmenge von w-3-Fettsäuren mindestens 20 Volumenprozent beträgt; und
(c) einen Geschmacksträger in einer Menge von 10 bis 89,5 Volumenprozent.

2. Nahrungsergänzungsmittel oder Functional Food nach Anspruch 1, wobei das Nussöl Mandelöl, Haselnussöl oder Walnussöl ist.

3. Nahrungsergänzungsmittel oder Functional Food nach Anspruch 1 oder 2, wobei die ω-3-fettsäurenhaltige Quelle ein Öl ist.

4. Nahrungsergänzungsmittel oder Functional Food nach Anspruch 3, wobei das Öl ein Fischöl, Algenöl oder ein aus Planktonmasse hergestelltes Öl ist wobei die Planktonmasse vorzugsweise im wesentlichen aus Crypthecodinium cohnii besteht.

5. Nahrungsergänzungsmittel oder Functional Food nach einem der Ansprüche 1 bis 4, wobei die ω-3-Fettsäuren der Quelle α-Linolensäure, Eicosapentaensäure und/oder Docosahexaensäure ist/sind.

6. Nahrungsergänzungsmittel oder Functional Food nach einem der Ansprüche 1 bis 5, wobei der Geschmacksträger ein Pflanzenöl ist.

7. Nahrungsergänzungsmittel oder Functional Food nach Anspruch 6, wobei das Pflanzenöl Olivenöl, Rapsöl, Leinöl, Sojaöl, Weizenkeimöl, Haselnussöl, Walnussöl oder Mandelöl ist.

8. Nahrungsergänzungsmittel oder Functional Food nach einem der Ansprüche 1 bis 7, weiterhin enthaltend (d) ein Antioxidans.

9. Nahrungsergänzungsmittel oder Functional Food nach Anspruch 8, wobei das Antioxidans Vitamin E und/oder Vitamin C ist.

10. Speiseöl bestehend aus oder enthaltend das Nahrungsergänzungsmittel oder Functional Food oder Functional Food nach einem der Ansprüche 1 bis 9.

11. Speiseöl nach Anspruch 10, das ein Salatöl ist.

12. Verwendung des Nahrungsergänzungsmittels oder Functional Food nach einem der Ansprüche 1 bis 9 als Speiseöl.

13. Verwendung nach Anspruch 12, wobei das Speiseöl ein Salatöl ist.
